Europäisches Patentamt

European Patent Office

Office européen des brevets

(19)

(11) Numéro de publication: **0 242 264**
A1

(12) **DEMANDE DE BREVET EUROPEEN**

(21) Numéro de dépôt: 87400747.9

(22) Date de dépôt: 03.04.87

(51) Int. Cl.⁴: **C 12 P 19/34**
C 12 N 15/00, C 12 Q 1/68,
C 12 Q 1/70

(30) Priorité: 04.04.86 FR 8604913

(43) Date de publication de la demande:
21.10.87 Bulletin 87/43

(84) Etats contractants désignés:
AT BE CH DE ES GB GR IT LI LU NL SE

(71) Demandeur: INSTITUT PASTEUR
25-28, rue du Docteur Roux
F-75724 Paris Cédex 15 (FR)

CENTRE NATIONAL DE LA RECHERCHE SCIENTIFIQUE
(CNRS)
15, Quai Anatole France
F-75007 Paris (FR)

(72) Inventeur: Avrameas, Stratis
1, rue Sarasate
F-75015 Paris (FR)

Sakamoto, Hiroshi
3, sentier des Jardies
F-92190 Meudon (FR)

Traincard, Francois
6, rue Dombasle
F-75015 Paris (FR)

Vo Quang, Tuyen
5 Villa Croix Nivert
F-75015 Paris (FR)

Guesdon, Jean-Luc
12 rue du Hameau
F-75015 Paris (FR)

Ter Nynck, Thérèse
39 bld Garibaldi
F-75015 Paris (FR)

(74) Mandataire: Gutmann, Ernest et al
S.C. Ernest Gutmann - Yves Plasseraud 67, boulevard
Haussmann
F-75008 Paris (FR)

(54) Sonde monocaténaire marquée, non radioactive, procédé pour sa fabrication et procédé de détection d'une séquence nucléotidique déterminée à l'aide de cette sonde.

(57) L'invention concerne une nouvelle sonde de détection d'une séquence nucléique déterminée. Elle consiste dans un ADN recombinant contenant un insérat complémentaire de la séquence nucléique à détecter et des séquences nucléiques dérivées de l'ADN (+) du phage M13, les bases entrant dans la composition de cet ADN recombinant, notamment les groupes thymine étant remplacés par des groupes 5-bromo-uracile. L'invention concerne également un procédé pour la fabrication de cette sonde, ce procédé comprenant la culture d'hôtes cellulaires, plus particulièrement E. coli, auxotrophes pour la thymine, dans un milieu contenant une teneur limitante en thymine et du 5-bromo-uracile. Après le temps d'incubation requis, on élimine le surnageant et on récupère la sonde à partir des cellules bactériennes.

EP 0 242 264 A1

**Description**

Sonde monocaténaire marquée, non radioactive, procédé pour sa fabrication et procédé de détection d'une séquence nucléotidique déterminée à l'aide de cette sonde

L'invention est relative à une sonde spécifique marquée non radioactive, monocaténaire, et à un procédé pour la détection par hybridation de la présence et, éventuellement, de la caractérisation d'une séquence nucléotidique déterminée au sein d'une composition contenant des acides nucléiques. Cette sonde marquée non radioactive contient elle-même une séquence nucléotidique complémentaire de la séquence nucléotidique à détecter. L'essai de détection implique par conséquent une réaction d'hybridation, dans les conditions appropriées, entre la séquence complémentaire portée par la sonde et la séquence nucléotidique déterminée à détecter, généralement en présence d'acides nucléiques autres que ceux contenant la séquence à détecter.

L'invention est également relative à un procédé pour la fabrication d'une telle sonde non radioactive.

Les sondes d'hybridation spécifiques pour détecter une séquence nucléique déterminée (ADN ou ARN) ont longtemps été - et sont encore souvent - marquées avec des isotopes radioactifs permettant une détection autoradiographique.

Les principaux inconvénients de la détection autoradiographique de l'hybridation sont bien connus. Ils résident essentiellement dans les dangers encourus par l'expérimentateur lorsqu'il manipule des produits radioactifs, le faible pouvoir de résolution, l'imprécision de la localisation, la longueur du temps d'exposition des films (de quelques heures à plusieurs semaines) et l'instabilité des sondes dues à la décomposition radiolytique et à la période propre des radioisotopes utilisés.

Pour remédier à cette difficulté, il a déjà été proposé de remplacer le marquage radioactif par d'autres modes de marquage. On rappellera en particulier la technique décrite pour la première fois dans le brevet francais no 78 10975 déposé le 13 avril 1978.

Le procédé décrit dans le brevet sus-visé met en jeu une sonde modifiée par couplage ou en vue de son couplage - direct ou indirect - avec une enzyme, de préférence postérieurement à la réaction d'hybridation, l'éventuelle présence de la séquence d'acide nucléique ou de l'acide nucléique recherché étant ensuite révélable par l'action du produit de couplage de l'enzyme et de la sonde (alors hybridée avec la séquence nucléique recherchée) sur un substrat de l'enzyme. A titre d'exemple d'enzyme couramment utilisée, on mentionnera la bêta-galactosidase, la gluco-oxydase, la phosphatase alcaline, les péroxydases. En variante, la détection ou la "révélation" de la sonde hybridée peut mettre en oeuvre des moyens distincts de l'enzyme, par exemple des techniques d'immunofluorescence.

Pour effectuer la modification de la sonde en vue de son couplage ultérieur avec une enzyme ou une molécule immunofluorescente, on a de plus en plus recours à l'incorporation dans l'acide nucléique de la sonde de nucléotides porteurs d'haptènes susceptibles d'être reconnus par des anticorps déterminés. L'opération de détection proprement dite met alors souvent en jeu une séquence de réactions comprenant une première mise en contact de la sonde hybridée porteuse des haptènes avec les anticorps anti-haptène, puis une seconde mise en contact de la sonde hybridée qui retient alors des anticorps anti-haptène, avec des immunoglobulines ou autres molécules affines dirigées contre ces anticorps, ces immunoglobulines portant elles-mêmes un marqueur non radioactif, par exemple enzymatique ou fluorescent. Il n'est pas besoin d'insister sur les conditions dans lesquelles ces techniques peuvent être mises en oeuvre. On se reportera par exemple à la demande de brevet européen no 0063869-A3, dans laquelle plusieurs de ces techniques sont décrites.

Les techniques qui viennent d'être rappelées et qui mettent toutes deux en oeuvre des sondes non radioactives ou, pour la commodité du langage ci-après des "sondes froides" (par oppositiion aux "sondes chaudes" que constituent les sondes marquées avec des isotopes radioactifs), présentent des avantages importants par rapport aux "sondes chaudes", ne serait-ce qu'au niveau de la plus grande sécurité des expérimentateurs qui les mettent en oeuvre. Mais il est toujours encore nécessaire de perfectionner les sondes elles-mêmes pour les rendre à la fois plus efficaces et plus sensibles, d'une part, ainsi que les procédés pour les fabriquer, afin de réduire le coût de ces sondes.

Pour tenter d'atteindre ces objectifs, il a déjà éé proposé, notamment dans la demande de brevet européen no 0 063 869-A3 déjà mentionnée et dans la demande de brevet européen no 0 143 059-A1 déposée le 23 novembre 1984, de modifier une partie des bases contenues dans l'un des nucléotides constitutifs de la sonde par des groupes de substitution de faible importance moléculaire, notamment en position 5, lorsqu'il s'agit d'une base pyrimidine et en position 7 ou en position 8, ou dans les deux positions à la fois, d'une base déazapurine ou purine.

D'une façon générale, on peut mettre en oeuvre toute base modifiée qui n'interfère pas avec la capacité d'appariement WATSON-CRICK de l'acide nucléique ainsi modifié avec un acide nucléique complémentaire naturel ou contenant des bases modifiées semblables. Mais les sondes préférées sont celles dans lesquelles les nucléosides modifiés sont des groupes 5-halogéno-uridine ou 5-halogéno-déoxy-uridine, et plus particulièrement encore celles dans lesquelles ces nucléosides modifiés sont des groupes 5-bromo-uridine ou 5-bromo-déoxy-uridine. d'autres groupes préférés susceptibles d'être mis en oeuvre dans le cadre de cette invention font intervenir des dérivés correspondants substitués par de l'iode ou du fluor, ou encore par des groupes $CF_3$.

Ces sondes froides peuvent alors, après hybridation, être détectées au moyen d'anticorps, de préférence des anticorps monoclonaux dirigés sélectivement contre les bases halogénées.

La demande de brevet européen nº 0 143 059-A1 susmentionnée décrit également un procédé pour la fabrication in vivo de sondes du genre sus-défini dans des micro-organismes, notamment des bctéries, et plus particulièrement E. coli, à partir d'un ADN recombinant ou d'un ADN-vecteur apte à transformer un micro-organisme, tel que plasmide, cosmide ou ADN de phage, dans lequel avait auparavant été insérée la séquence nucléotidique complémentaire (ci-après appelée insérat) de la séquence d'acide nucléique à détecter. Ce procédé comprend la mise et le maintien en culture du micro-organisme préalablement transformé par cet ADN recombinant avec un milieu de culture contenant la base modifiée en remplacement de la base naturelle correspondante, la susdite base modifiée s'y trouvant, soit à l'état libre, soit à l'état combiné, dans un nucléoside ou nucléotide correspondant à ladite base, et par la récupération ultérieure de l'ADN-recombinant ou ADN vecteur.

Ce procédé permet alors la substitution in vivo de l'une au moins des bases contenues à l'intérieur de ces ADN-recombinants et ADN-vecteurs au sein même du micro-organisme maintenu en culture dans le milieu contenant la base modifiée.

La présente invention a pour but l'obtention de sondes froides recombinantes, encore plus efficaces, présentant un degré de sensibilité au niveau de la détection jamais encore atteint avec ce type de sondes marquées in vivo.

Elle a encore pour but un procédé de fabrication in vivo particulièrement simple de ces sondes, après transformation d'un micro-organisme approprié, notamment E. coli, par un vecteur recombinant contenant un insérat spécifique de la séquence nucléotidique à détecter.

L'invention repose sur un choix particulier de séquences d'ADN qui, en combinaison avec l'insérat spécifique, vont constituer la sonde froide plus particulièrement décrite et revendiquée ci-après.

La sonde recombinante selon l'invention est caractérisée :
- par son caractère monocaténaire de polarité unique non autohybridable,
- par l'association de l'insérat spécifique de la séquence nucléotidique à détecter, avec des brins hétérologues présentant des séquences de nucléotides contenues dans l'ADN d'un phage à brin unique, dont le cycle biologique infectieux à l'égard de l'hôte cellulaire compétent, implique normalement la production d'une matrice double-brin intracellulaire retenue à l'intérieur de l'hôte cellulaire et l'excrétion du brin unique de réplication, notamment sous forme encapsidée, dans le milieu de culture du micro-organisme compétent et
- par un groupe de modification sur la base de l'un des nucléotides constitutifs de l'ensemble de la sonde par un groupe halogéné de substitution, et ce à raison de plus de 60 %, de préférence de la totalité des nucléotides concernés dans ladite sonde, ladite modification rendant la sonde reconnaissable par des anticorps spécifiques reconnaissant eux-mêmes ce groupe de modification.

Avantageusement les brins associés avec la séquence d'insertion correspondant à des séquences d'ADN monocaténaires contenues dans le génome d'un phage, infectieux à l'égard de E. coli, du type de ceux qui sont connus sous la désignation M13, selon la définition qu'en donne par exemple le brevet français nº 82 18469/2.519.094.

Le procédé selon l'invention pour produire la sonde recombinante susdite est du genre de ceux qui comprennent une étape d'infection d'un hôte cellulaire compétent par un vecteur contenant l'insérat spécifique de la sonde dans l'une de ses régions non essentielles eu égard à ses facultés de réplication dans cet hôte cellulaire, une étape de propagation de l'hôte cellulaire dans un milieu de culture approprié et une étape de récupération de la sonde.

Le procédé selon l'invention est plus particulièrement caractérisé par la combinaison des caractéristiques suivantes :
- le vecteur est un phage choisi parmi les phages à brin unique dont le cycle biologique infectieux à l'égard de l'hôte cellulaire compétent correspondant implique normalement la production d'une matrice double brin-intracellulaire retenue à l'intérieur de l'hôte cellulaire et l'excrétion des brins uniques répliqués, notamment sous formes encapsidées, dans le milieu de culture,
- l'hôte cellulaire est auxotrophe pour la base naturelle entrant dans la constitution de l'un des quatre nucléotides naturels,
- le milieu dans lequel est réalisée la propagation de l'hôte cellulaire a une teneur limitante en ladite base naturelle,
- le milieu contient aussi, du moins au moment où la base naturelle a été entièrement consommée, une teneur en une base correspondant à la susdite base, cette base étant cependant modifiée par un groupe halogéné,
- on maintient la culture à partir du moment où la base naturelle a été entièrement consommée pendant une durée sufffisante à assurer l'excrétion de l'ADN viral répliqué non modifié dans le milieu de culture ainsi que l'accumulation de l'ADN viral répliqué modifié dans l'hôte cellulaire,
- on récupère ensuite l'hôte cellulaire dont on extrait alors l'ADN viral monocaténaire contenant les bases modifiées.

Lorsque l'on utilise un vecteur viral du type M13, l'hôte cellulaire compétent préféré est constitué par un E. coli auxotrophe pour l'une des quatre baes entrant dans la constitution des nucléotides, de préférence pour la thymine.

Le groupe halogéné de modification auquel référence est faite dans les définitions de la sonde et du procédé selon l'invention est constitué par tout groupe de ce type qui n'interfère pas avec la capacité du vecteur modifié à être reconnu par les polymérases de l'hôte cellulaire compétent et, ultérieurement, avec la capacité de la sonde à s'hybrider avec la séquence nucléique recherchée. Des nucléosides modifiés entrant dans la constitution des sondes selon l'invention peuvent être ceux qui ont été rappelés plus haut, en rapport avec l'invention décrite dans la demande de brevet européen n°0 143 059-A1.

En ce que concerne plus particulièrement le procédé selon l'invention, la base modifiée utilisée dans l'étape correspondante peut être constituée soit par la base elle-même à l'état libre, soit à l'état combiné dans le nucléoside ou le nucléotide correspondant à ladite base. De préférence, la modification affecte les bases thymine. Les nucléosides correspondants dans la sonde sont avantageusement constitués par des groupes 5-halogénouridine ou 5-halogéno-déoxy-uridine, et plus particulièrement encore ceux dans lesquels ces bases modifiées sont des groupes 5-bromo-uridine ou 5-bromo-déoxy-uridine.

Les sondes froides selon l'invention ont la particularité d'être monocaténaires, de polarité unique et non-autohybridables. Ces caractéristiques comptent eu égard à la très grande sensibilité de détection qui peut être atteinte avec ces sondes. En outre, le caractère monocaténaire assure un accès optimum aux molécules affines, notamment anticorps monoclonaux reconnaissant les bases modifiées. Cette observation s'étend particulièrement aux anticorps monoclonaux capables de reconnaître des bases brominées, telles que les groupes 5-halogénouracile, plus particulièrement 5-bromouracile.

L'intérêt particulier de la sélection du vecteur utilisé parmi les nombreux vecteurs utilisables réside dans la découverte faite par les inventeurs que la modification de certaines des bases dans les produits de réplication de l'ADN génomique des phages considérés à l'intérieur des hôtes cellulaires correspondants, les rendait inaptes à franchir les membranes cellulaires de l'hôte, à l'opposé de ce que l'on observe avec les ADNs de réplication correspondants, non modifiés, obtenus à partir des mêmes phages. Cette caractéristique se manifeste par plusieurs avantages spécifiques.

Elle permet la séparation au sein même de l'hôte cellulaire des ADNs de réplication non modifiés (excrétés dans le milieu de culture) et des ADNs de réplication modifiés, retenus au sein de l'hôte cellulaire. En outre, surtout dès lors que les conditions du procédé de fabrication sont convenablement réglés, il est possible d'aboutir à une substitution complète de la base concernée dans les ADNs de réplication par la base modifiée correspondante. Enfin ces ADNs monocaténaires modifiés, contenant la séquence d'insertion caractéristique de la sonde, peuvent être obtenus à partir de l'hôte cellulaire, préalablement séparé du milieu de culture. Après rupture des parois bactériennes, la sonde modifiée est directement accessible par des procédés de purification du type de ceux applicables à la séparation, par exemple par centrifugation différentielle dans un gradient approprié, de plasmides à partir d'extraits cytoplasmiques des cellules qui les hébergent. On notera que ces phages modifiés, contenant la séquence d'insertion spécifique de la sonde, sont obtenus directement à l'état monocaténaire, en l'absence de toute protéine d'encapsidation. Il résulte donc de ce qui précède, que la principale différence entre le protocole classique de récupération des ADNs de réplication des phages du genre en question et la méthode de l'invention, réside dans le fait que dans le premier cas l'ADN de réplication est obtenu à partir du surnageant (l'ADN monocaténaire étant alors généralement à l'état encapsidé), alors que dans le cas du procédé selon l'invention, l'ADN modifié contenant la séquence d'insertion est obtenu à partir des hôtes cellulaires eux-mêmes.

Ainsi qu'il a été dit plus haut, l'hôte cellulaire compétent préféré, en l'occurrence E. coli, lorsque l'on utilise un phage du type M13 ou analogue, est auxotrophe pour l'une des quatre bases, par exemple la thymine, entrant dans la constitution des nucléotides, et le milieu dans lequel est réalisée la propagation de l'hôte cellulaire a une teneur limitante en la base naturelle correspondante.

Par l'expression "teneur limitante", il faut ici entendre une teneur initiale en bases naturelles, par exemple la thymine, inférieure à la concentration de la base naturelle susceptible d'être absorbée par la culture cellulaire transformée pour atteindre son maximum de développement. En d'autres termes, cette teneur limitante a pour effet de bloquer le développement de la culture à un niveau en deçà de sa capacité de développement maximum, en raison de l'auxotrophie des cellules qui la composent vis-à-vis de la base naturelle. Cependant, si le milieu de culture contient également la base modifiée correspondante au moment où la teneur en base naturelle s'épuise, l'activité cellulaire se poursuit, même si la culture ne tend plus à se développer, et ce en présence de la base modifée correspondante. C'est dans cette phase de post-croissance que s'élaborent alors les ADNs viraux contenant la base modifiée et qui constitueront ultérieurement les sondes conformes à l'invention. Au cours de cette activité cellulaire, les ADNs de réplication encore formés avec la base naturelle sont excrétés dans le milieu de culture, tandis que les ADNs viraux de réplication formés avec la base modifiée restent emprisonnés à l'intérieur des enveloppes que définissent les membranes cellulaires.

Avantageusement, la teneur limitante correspond à la teneur en bases naturelles qui correspond à celle qui est requise pour atteindre la vitesse d'accroissement maximum de culture par unité de temps. A cet instant l'activité cellulaire est alors au maximum, avec pour conséquence la production de quantités importantes d'ADNs viraux de réplication contenant lesdites bases modifiées, par les cellules qui cependant cessent de se diviser en raison de la disparition de la base naturelle.

Des mutants de E. coli, auxotrophes pour la thymine, peuvent être sélectionnés en présence de triméthoprine, selon la technique décrite par Miller et al, 1972, "Experiments in Molecular Genetics", Cold Spring Harbor Laboratory, ou encore selon le protocole décrit par Stacey K. A. et al,(1965), J. Bact. 90, 554-555.

On peut naturellement utiliser d'autres types de bases modifiées, par exemple la 5-bromocytosine, la 8-bromo-guanine et la 8-bromo-adénine. On se reportera cependant à la demande de brevet européen n° 0 143 059-A1, pour ce qui est des contraintes supplémentaires auxquelles doivent répondre les mutants de E. coli utilisés. Ces mutants devront également comporter par exemple des mutations dans les gènes codant pour la ribonucléoside diphosphate-réductase (EC 1.17.4.1), notamment au niveau des loci nrdA et nrdB (Bachmann et al, 1980, Microbiological Reviews, 44: 1-56).

On comprendra que la base modifiée peut être présente dans le milieu de culture dès l'origine ou ajoutée à tout instant au milieu de culture avant que sa teneur en bases naturelles ne soit épuisée.

L'ensemble des opérations qui précèdent peut également être réalisé à l'abri des radiations ayant des longueurs d'ondes inférieures à 400 nanomètres (ces radiations pouvant entraîner la perte de l'halogène dans le cas d'acides nucléiques modifiés par des groupes 5-halogéno-uracile). Notamment la préparation des sondes selon l'invention in vivo et in vitro peut être réalisée à l'obscurité ou à une lumière constituée de radiations ayant des longueurs d'onde supérieures à 400 nanomètres. L'introduction dans le milieu de culture des hôtes transformés par le vecteur porteur de l'acide nucléique (insérat) destiné à la préparation d'une sonde, d'un colorant inerte, non toxique pour l'hôte et le vecteur et absorbant dans les longueurs d'ondes inférieures à 400 nanomètres, peut constituer un moyen permettant de s'affranchir de la contrainte que pourraient imposer, dans certains cas, les restrictions qui précèdent quant à la nature de la lumière à utiliser.

Les sondes selon l'invention peuvent être conservées à l'état lyophilisé danstout récipient ou sur tout support adéquats. Ils peuvent également être conservés dans des flacons ou récipients faits en une matière absorbant dans les longueurs d'ondes inférieures à 400 nanomètres. Les sondes selon l'invention peuvent également être conservées à l'état de solutions, soit dans un récipient répondant aux conditions sus-indiquées, soit dans des récipients dont les parois ne sont pas absorbantes, cas dans lequel ces solutions contiennent elles-mêmes un colorant inerte absorbant dans les longueurs d'ondes inférieures à 400 nm.

Des caractéristiques supplémentaires de l'invention apparaîtront encore au cours de la description qui suit d'un exemple de production d'une sonde froide monocaténaire conforme à l'invention, étant entendu que cette description ne saurait être interprétée comme ayant un caractère limitatif quant à la portée des revendications de la présente demande de brevet.

EXEMPLE :
Le phage utilisé est constitué par le bactériophage M13. Il est identique à celui qui se trouve être contenu dans le micro-organisme transformé E. coli JM103(W14) qui a été décrit dans la demande de brevet français n°82 18469/2.516.094. Un tel phage est par exemple accessible à la "National Collection of Industrial Bacteria" (N.C.I.B.) de la Torry Research Station, Aberdeen, Ecosse. Il y a été déposé le 12 novembre 1981. Il a reçu le numéro d'admission 11.704.

C'est aussi la souche JM103 (décrite dans l'article de Maniatis T. et al, Cold Spring Harbor, 1982) qui a été utilisée comme souche de base dans le cadre de l'invention. Une sélection d'une souche auxotrophe pour la thymine a été réalisée dans les conditions rappelées plus haut. Le clone sélectionné, dénommé TUC0701, s'est révélé être un mutant thymine-dépendant nécessitant au moins 50 microgrammes/millilitre de thymine pour une croissance optimale en milieu synthétique liquide, non mucoïde, permettant la croissance du bactériophage M13. Cette souche a été déposée à la Collection Nationale de Culture de Microorganismes (C.N.C.M.) de l'Institut Pasteur de Paris le 3 avril 1986. Le numéro de dépôt suivant lui a été accordé : I-541.

Cette souche a été utilisée pour incorporer in vivo la 5-bromo-désoxy-uridine dans l'ADN du bactériophage M13 préalablement modifié dans une de ses régions non essentielles pour sa réplication par un insérat nucléotidique spécifique complémentaire de la séquence nucléotidique à détecter. Une telle sonde modifiée peut être réalisée en mettant en oeuvre le protocole de séparation décrit ci-après pour le marquage de l'ADN (plus particulièrement du brin +) de l'ADN du phage M13.

On a préparé 2 ml de préculture de la souche TUCO701. Celle-ci a été cultivée à 37°C pendant la nuit, en milieu synthétique M9, complétée avec de la thymine pour atteindre une concentration finale de 0,1 mg/ml.

2 ml d'un stock de bactériophages M13 sont ajoutés au milieu.

Le mélange est incubé pendant 15 minutes à 37°C en l'absence d'agitation.

On ajoute alors 100 ml de milieu riche en nutriment : 2 x YT additionné de 5-bromo-uracile à raison de 0,3 mg/ml final.

On cultive à 37°C sous agitation vigoureuse, le cas échéant avec oxygénation à l'air comprimé et régulation du pH à la valeur 7.

La courbe de croissance de la culture est observée (par mesure de l'évolution de la densité optique à 600 nanomètres). Les bactéries sont récoltées 2 heures après le début de la phase stationnaire. Le surnageant, qui contient éventuellement des ADNs viraux encapsidés, est écarté.

Lorsque la susdite séparation est réalisée par centrifugation, c'est le culot bactérien qui constitue par conséquent la matière première pour l'extraction ultérieure de la sonde. En particulier, après lyse des bactéries, on extrait l'ADN extra-chromosomique et on le purifie par la méthode dite de chromatographie rapide de protéines en phase liquide, connue sous l'abréviation FPLC (de l'anglais "Fast Protein Liquid Chromatograophy"), par exemple selon la méthode décrite par Vo-Quang T. et al, Bioscience Reports (1985) 5, 101-111 ou par toute autre technique adéquate, par exemple selon la technique de Maniatis T. et al, "Molecular Cloning" (Clonage Moléculaire), Cold spring harbor Laboratory, 1982.

L'ADN de réplication du phage M13, modifié par les groupes 5-bromo-uracile, peut, lorsqu'il contient également l'insérat spécifique susdit, être directement utilisé comme sonde, puisqu'il est déjà débarrassé de la capside du phage. Elle doit se trouver à l'état monocaténaire à au moins 90 % (la proportion de sonde marquée bicaténaire est mesurable, après traitement de la sonde marquée à la nucléase SI) (Maniatis et al).

La technique décrite ci-dessus permet des rendements de production très élevés : au moins 10 microgrammes de sonde marquée par millilitre de culture bactérienne, soit un rendement du même ordre de grandeur que celui obtenu dans les protocoles classiques d'extraction de l'ADN monocaténaire non marqué du bactériophage M13, notamment dans les conditions décrites par la société qui le commercialise : Amersham "M13 Cloning and Sequencing Kit" (Kit de clonage et de séquençage).

Les sondes marquées obtenues se caractérisent par une très grande sensibilité. Le seuil de détection testé par la méthode dite du "test de tache" (dot test) décrite par Traincard F. et al Ann. Immuno. Inst. Pasteur (1983) 134 D 399-405 se situe à 1pg de sonde déposée (révélation par la péroxydase/diaminobenzidine).

Les sondes obtenues peuvent être utilisées de toute façon en soi connue. Si l'on a recours à la technique d'hybridation par taches (dot hybridization) décrite par Traincard F. et al déjà cités, on constate que les sondes selon l'invention permettent de détecter un pg d'ADN-cible déposé sur une membrane, étant entendu que l'expression "ADN-cible" se réfère à l'ADN complémentaire à la sonde (révélation par la phosphatase alcaline/bromochloro-indolyl-phosphate/nitro bleu tétrazolium).

Les sondes obtenues sont uniformément et totalement marquées. Ce résultat n'était réalisable dans le cadre des techniques antérieures que dans des systèmes de marquage beaucoup plus complexes.

Les milieux de culture M9 et 2 x TY sont décrits dans la notice publiée par Amersham déjà cité (M13 Cloning and Sequencing Kit).

Il va naturellement de soi que l'invention concerne toutes les variantes possibles. Elle a été plus particulièrement décrite en rapport avec le phage M13. Il va sans dire qu'elle peut être appliquée dans des conditions semblables avec d'autres phages répondant aux conditions qui ont été définies plus haut. A titre d'exemple d'autres phages utilisables dans des conditions semblables, on utilisera celui connu sous la désignation φX174.

Les sondes ainsi modifiées peuvent être utilisées dans des essais de détection par hybridation dans des conditions classiques. Avantageusement la détection comprend, postérieurement à l'opération d'hybridation, une étape de reconnaissance de la sonde hybridée par l'intermédiaire d'anticorps monoclonaux dirigés contre la base modifiée.

Pour ce qui est des techniques, on peut se reporter à titre d'exemples à la description de la demande de brevet européen n° 0 143 059-A1.

Il va de soi que les diverses publications et demandes de brevet qui ont été mentionnées dans le cadre de la présente description doivent être considérées comme en faisant partie, dès lors que les précisions qu'elles contiennent peuvent servir à éclairer la signification de certains des éléments caractéristiques de la présente invention.

**Revendications**

1 - Sonde recombinante caractérisée :
- par son caractère monocaténaire, de polarité unique, non autohybridable,
- par l'association de l'insérat spécifique de la séquence nucléotidique à détecter, avec des brins hétérologues présentant des séquences de nucléotides contenues dans l'ADN d'un phage à brin unique, dont le cycle biologique infectieux à l'égard de l'hôte cellulaire compétent, implique normalement la production d'une matrice double-brin intracellulaire retenue à l'intérieur de l'hôte cellulaire et l'excrétion du brin unique de réplication, notamment sous forme encapsidée, dans le milieu de culture du micro-organisme compétent et
- par un groupe de modification sur la base de l'un des nucléotides constitutifs de l'ensemble de la sonde par un groupe halogéné de substitution, et ce à raison de plus de 60 %, de préférence de la totalité des nucléotides concernés dans ladite sonde, ladite modification rendant la sonde reconnaissable par des anticorps spécifiques reconnaissant eux-mêmes ce groupe de modification.

2 - Sonde recombinante selon la revendication 1, caractérisée en ce que les brins hétérologues associés avec l'insérat spécifique présente des séquences de nucléotides contenues dans l'ADN d'un phage M13, si ce n'est la modification de la base de l'un des quatre nucléotides entrant dans la constitution de la sonde.

3 - Sonde selon la revendication 1 ou la revendication 2, caractérisée en ce que les bases modifiées sont dérivées de la thymine, notamment consistant en des groupes 5-bromo-uracile.

4 - Sonde selon l'une quelconque des revendications 1 à 3, caractérisée en ce que la modification affecte la totalité des bases concernées.

5 - Procédé de fabrication d'une sonde conforme à l'une quelconque des revendications 1 à 4, qui comprend une étape d'infection d'un hôte cellulaire compétent par un vecteur contenant l'insérat spécifique de la sonde dans l'une de ses régions non essentielles eu égard à ses facultés de réplication dans cet hôte cellulaire, une étape de propagation de l'hôte cellulaire dans un milieu de culture approprié et une étape de récupération de la sonde, ce procédé étant caractérisé par la combinaison des caractéristiques suivantes :
- le vecteur est un phage choisi parmi les phages à brin unique dont le cycle biologique infectieux à l'égard de l'hôte cellulaire compétent correspondant implique normalement la production d'une matrice double brin-intracellulaire retenue à l'intérieur de l'hôte cellulaire et l'excrétion des brins uniques répliqués, notamment sous formes encapsidées, dans le milieu de culture,

- l'hôte cellulaire est auxotrophe pour la base naturelle entrant dans la constitution de l'un des quatre nucléotides naturels,

- le milieu dans lequel est réalisée la propagation de l'hôte cellulaire a une teneur limitante en ladite base naturelle,

- le milieu contient aussi, du moins au moment où la base naturelle a été entièrement consommée, une teneur en une base correspondant à la susdite base, cette base étant cependant modifiée par un groupe halogéné,

- on maintient la culture à partir du moment où la base naturelle a été entièrement consommée pendant une durée sufffisante à assurer l'excrétion de l'ADN viral répliqué non modifié dans le milieu de culture ainsi que l'accumulation de l'adn viral réplilqué modifié dans l'hôte cellulaire,

- on écarte le surnageant du milieu de culture et on récupère l'hôte cellulaire dont on extrait alors l'ADN viral monocaténaire contenant les bases modifiées.

6 - Procédé selon la revendication 5, caractérisé en ce que l'hôte cellulaire compétent est un mutant de E. coli et en ce que le phage est un bactériophage du type M13.

7 - Procédé selon la revendication 5 ou la revendication 6, caractérisé en ce que l'hôte cellulaire est auxotrophe pour la thymine et en ce que la base modifiée présente dans le milieu de culture lorsque sa teneur en base naturelle est épuisée, est constitué par le 5-bromouracyle.

8 - Procédé de détection d'une séquence nucléotidique au sein d'un acide nucléique, notamment en présence d'autres acides nucléiques, caractérisé par la mise en contact de cette séquence nucléique avec une sonde conforme à l'une quelconque des revendications 1 à 4 dans des conditions autorisant l'hybridation, puis la détection de la sonde hybridée par une molécule ou une composition ayant une affinité spécifique pour le groupe de modifications entrant dans la composition des bases modifiées de la sonde.

9 - Procédé selon la revendication 8, caractérisé en ce que la composition affine est constituée par des anticorps monoclonaux reconnaissant spécifiquement ce groupe de modification.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 133 473 (ENZO BIOCHEM, INC.)<br>* Abrégé; exemple III; revendications 1-3,14-17 * | 1-9 | C 12 P 19/34<br>C 12 N 15/00<br>C 12 Q 1/68<br>C 12 Q 1/70 |
| | --- | | |
| D,A | EP-A-0 143 059 (INSTITUT PASTEUR)<br>* En entier * | 1-9 | |
| | --- | | |
| A | CHEMICAL ABSTRACTS, vol. 102, no. 17, 29 avril 1985, page 148, résumé no. 144114n, Columbus, Ohio, US; H. SEO et al.: "M13 cloning and its application for the synthesis of a single-stranded hybridization probe" & ENVIRON. MED. 1984, 28, 41-5<br>* Résumé * | 1-6 | |
| | ---     -/- | | DOMAINES TECHNIQUES RECHERCHES (Int. Cl.4)<br><br>C 12 Q<br>C 12 N |

Le présent rapport de recherche a été établi pour toutes les revendications

| Lieu de la recherche | Date d'achèvement de la recherche | Examinateur |
|---|---|---|
| LA HAYE | 10-07-1987 | OSBORNE H.H. |

## DOCUMENTS CONSIDERES COMME PERTINENTS

Page 2

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 90, no. 9, 26 février 1979, pages 246-247, résumé no. 68974j, Columbus, Ohio, US; T.-C. CHEN et al.: "Replication of bacteriophage M13, XIV. Differential inhibition of the replication of M13 and M13 miniphage in a mutant of Escherichia coli defective in the 5' - 3' exonuclease associated with DNA polymerase I.", & J. VIROL. 1978, 28(3), 679-85<br>* Résumé *<br><br>----- | 1-6 | |
| | | | DOMAINES TECHNIQUES RECHERCHES (Int. Cl.4) |

Le présent rapport de recherche a été établi pour toutes les revendications

| Lieu de la recherche<br>LA HAYE | Date d'achèvement de la recherche<br>10-07-1987 | Examinateur<br>OSBORNE H.H. |
|---|---|---|